# EUROPEAN PATENT APPLICATION

(11) **EP 1 815 789 A1**
(43) Date of publication of application: **08.08.2007**
(21) Application number: 07002359.3
(22) Date of filing: 02.02.2007
(51) Int. Cl.: A61B 5/103, A63B 22/18

(54) **Improved system for diagnostic determination and consequent treatment of various pathologies by means of a combination of postural, pressure and photographic measurements**

(30) Priority: 03.02.2006 IT PN20060011
(71) Applicant: Cardone, Maurizio, 33080 Corva di Azzano Decimo PN (IT)
(72) Inventor: Cardone, Maurizio, 33080 Corva di Azzano Decimo PN (IT)
(74) Representative: Da Riva, Ermanno

(57) **Abstract**

Improved system for diagnostic determination and consequent treatment for various pathologies by combining postural pressure and photographic relevation means, it comprises a "baropodometric-stabilometric" platform (1) comprising the union of two platforms, i.e. a baropodometric platform and a stabilometric platform, as well as photographic cameras (2) positioned at right-angles, for frontal and lateral images of the patient (11), for static measurement of the data relating to the pathology of the patient (11), also characterized by the fact that the data measured by the "baropodometric-stabilometric" platform (1) and the photographic cameras (2) are then processed by a diagnostic processor (3) and then transmitted to the therapeutic elaborator (4), which then transmits the therapy to a "proprioceptive platform" (5) for the therapy.

## Description

The present invention has as objet an improved system for diagnostic determinations and the consequent therapy of various pathology referred to the optical, optometrical, odontostomatological, physiatrics and physiotherapy fielda and rehabilitative, as well as sports and aesthetic fields treatments realized bu a diagnostic system of postural, pressure and photographic measurements effects and a therapeutic system, all operating in interaction.

For diagnostics, the system involves the use of a system that combines - for measurement of the data relative to the pathology - a baropodometric-stabilometric platform with a photographic system for creating images. The data thus recorded are transmitted to an apparatus for diagnostic processing; after this apparatus has defined the diagnosis, the diagnostic data are transmitted to a therapeutic computer which determines the therapy for the treatment of the pathologies diagnosed, then sending the processed data to a second system comprising a proprioceptive platform which positions the patient in a posture that is appropriate for the treatment of the pathology diagnosed.

Applied systems in the various fields of diagnostics and therapeutics are widely known, and are used in diverse areas, such as rehabilitation of accident victims, or to treat pathologies such as scoliosis, cyrtosis, lordosis etc., but not for other specific therapeutic applications.

Methods of measurement and diagnosis generally consist of systems defined as "baropodometric platforms" and "stabilometric platforms". These platforms are used by podologists, orthopaedic technicians and, though rarely, by physiatrists solely to detect pressure dysfunctions in the sole of the foot.

Therapeutic methods generally comprise a tilting platform, and are used by the same physicians who utilize the diagnostic methods.

There follows a brief description of the methods used and their use for diagnostics and therapy, to this end highlighting the limitations of the traditional diagnostic and therapeutic system, i.e. baropodometry and stabilometry.

In detail: baropodometry is an evaluation of the pressure "peaks" for each foot, which are measured by a load cell. There exist two types of baropodometry: 1) static - 2) dynamic. Static baropodometry measures the pressure that the foot exerts on the load cell, thus providing a "footprint" of the force that the foot exerts. This procedure require approximately 20 seconds. In dynamic baropodometry, the dynamics of each step is analyzed. Dynamic analysis requires the patient to take a number of steps on a platform; the regularity of each single step is examined, comparing the left- and right-foot steps to ascertain their correct movement and symmetry. This type of examination provides a series of numerical data that quantify the status of the pathology of the patient solely from a podological standpoint. Stabilometry is a simple postural evaluation - in this case, too, concerning the sole of the foot - are is rarely used for a more complex examination of the condition of the patient. The stabilometric platform makes it possible to analyze the strategy of compensation and adaptation that a patient adopts in order to maintain a stable position and how to handle the state of constant unbalance that the patient suffers.

The stabilometric platform is used to measure the C.O.P. (centre of pressure). Interpolation of the pressures measured by each sensor (1,600 or more pressure measurements), provides various types of data. The line generated by the C.O.P., referred to as a "bundle", recorded during an acquisition period of approximately 52 seconds (the time during which a complete postural cycle takes place) makes it possible to determine the L.S.F. (the energy consumption index) and other parameters.

The methods described above are used separately, i.e. baropodometry is used first, followed by stabilometry for diagnosis.

After defining the diagnosis using these systems separately, a treatment can be prepared. The therapy indicated by the diagnostic systems described previously will be applied using an apparatus for proprioceptive rehabilitation, i.e. a tilting platform. The tilting platform serves as a rehabilitation tool that re-educates the zone of the brain that controls articulation and the correlated muscles, with the purpose of obtaining a more precise posture. This type of re-educative rehabilitation is found to be effective and long-lasting. The tilting platform can also be used with success in articular rehabilitation. The types of rehabilitation mentioned above may be summarized using two specific definitions relating to the sector: cortical rehabilitation, which relates to voluntary movements, and sub-cortical rehabilitation, which relates to involuntary movements of the subject undergoing therapy.

It seems that currently there exist no baropodometric and stabilometric platforms that are connected in any way with apparatus and/or systems for diagnosis; in addition, there exist no platform that operate at 360° and transmit data by radio to the diagnostic systems.

The market and the current techniques offer wooden boards on which the patient moves without any external feedback (external visible references); other types of board offer interaction with diagnostic systems using specific software, but the mobility of the platform is significantly reduced, to just 180° (mobility being defined as forward-backward movement or mediolateral movement). These do not move 360°, and are connected by wires for data transmission, which greatly increases the complexity of their structural and operation.

The systems described above are usually separate; in other words, the usual practise is to position the patient first on the baropodometric platform to measure the pressures exerted by the feet, and then to perform the stabilometric measurement; after these two phases have provided the values necessary for a diagnosis, the diagnosis is processed, after which the patient is placed on a tilting platform in order to assume the correct position.

The traditional method is laborious and frequently less than accurate, since the baropodometric and stabilometric values are measured separately and, when considered in combination, the results may not provide a correct perception of the pathology afflicting the patient. In addition, in the case of apparatus connected to diagnostic processors, the data transmitted via the wiring may not always be reliable for various reasons, such as contact resistances, voltage drops, etc.; this may result in signals with inaccurate voltage or amperage etc., and thus the diagnosis and the consequent therapy may be inaccurate and only approximate as regards a real condition of the patient.

In addition, the systems described above are limited in that they do not combine any photographic measurement simultaneously with the diagnostic measurement and therefore it is not possible to obtain parameters relating to the posture of the patient on the diagnostic platform, such as, for example, measurement of the angles or vertical of horizontal lines relative to the alignment of the patient, and the consequent treatment.

After definition of the diagnostic measurements, the data thus obtained will be processed and transmitted to a therapeutic system such as a tilting platform of a known type, but as described above these may not be precise enough to formulate a correct diagnosis and thus to indicate the appropriate therapy.

The purpose of this apparatus is to eliminate the inconvenience, the limitations and the problems outlined above, by creating the diagnostic/therapeutic system described below.

For a fuller understanding of the characteristics and the consequent advantages of using the therapeutic system which comprises this invention, the whole being if possible in a preferred form and merely as an exemplificative and non-limitative example, there follows a detailed description and claim referring to the attached schematic drawings, in which:
- Figure 1 is a block diagram illustrating a diagnostic/therapeutic system designed for static postural measurements;
- Figure 2 is a block diagram illustrating a supplementary variant: a dynamic diagnostic/therapeutic measurement system.

With particular reference to the figures mentioned above, common components are indicated by the same numerical references.

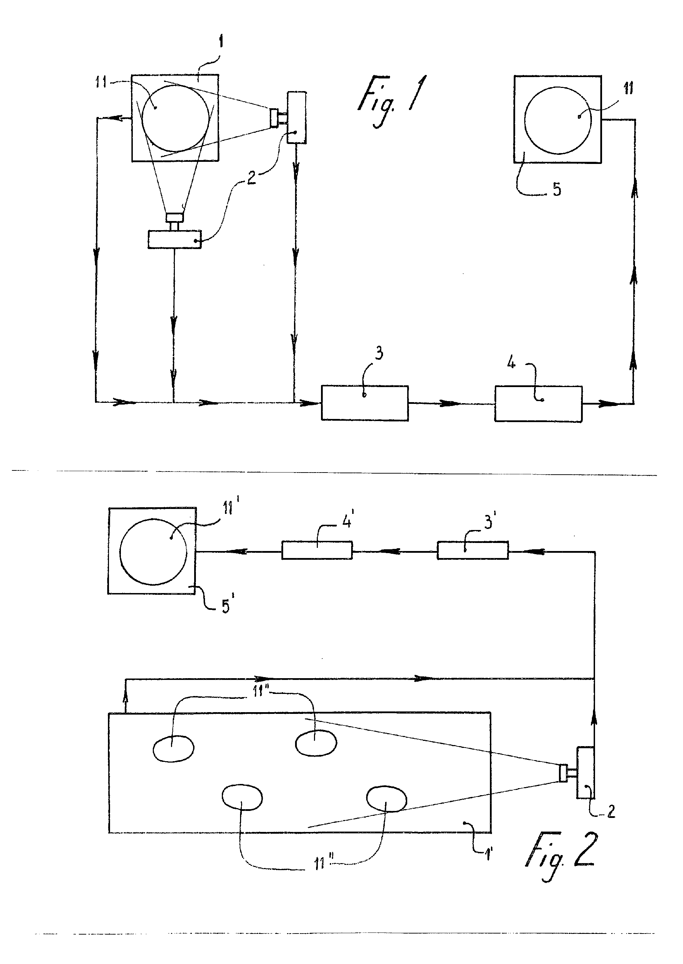

Figure 1 is a block diagram illustrating the complete static system which is the object of this invention and which is the basic system for diagnostics and therapy.

Primarily, figure 1 shows the diagnostic section in which detail 1 is a rectangle representing the first measurement system, which consists of a "baropodometric/stabilometric platform" upon which the patient (11) stands. On completion of the examination of the patient (11), as a second system, a photographic image is acquired using at least two camera (2) of the patient being diagnosed. This second system, a supplementary diagnostic technique, consists of at least two cameras placed at right-angles to each other and positioned as follows: the first camera (2) frontally, the second (2) laterally with respect to the patient (11). This system of photographic measurement, comprising cameras (2) positioned at right-angles to each other, makes it possible to determine the initial postural parameters of the patient. Thus we will obtain measurement of the pressure exerted and the evaluation of the patient's posture: the pressure exerted measured using the platform (1), the postural parameters using the cameras (2). All this data will subsequently be transmitted, as shown schematically by the arrows, to a diagnostic processor (3) (which may be a PC or a specific device) which will process the data received from elements 1 and 2. Thus the diagnostic processor (3) will output the values relative to the diagnosis of the pathology of the patient (1). Then, the data coming from the diagnostic processor (3) as described above are transmitted to a second, therapeutic processor (4) which processes the data to be sent to the tilting platform (5), which will place the patient in the most suitable position for the treatment of the pathology diagnosed.

The static system of detection of the pathology may be combined with a dynamic system (as shown in figure 2). The dynamic system comprises a platform (1') upon which the patient (11) is made to walk. This platform (1'), like platform 1, will determine the pressure exerted by the foot of the patient (11) and therefore any defects - whether dynamic or static - that might contribute to identify the real pathology of the patient; in addition to the system of dynamic measurement of the pressure exerted by the feet on platform 1', the apparatus offers a system of photographic measurement using at least one camera (2). As described above, dynamic baropodometry analyzes the dynamics of each step. This dynamic analysis requires the patient (11') to take a number of steps (11") on a platform 1', after which the regularity of each single step is examined by comparing the "right step" (11 ") with the "left step" (11 ") and verifying that these are correct and symmetrical.

From the procedure described above, the photographs of the steps (11 ") taken by the patient (11') on the platform (1'), obtained using the camera (2) will provide the criteria for the symmetry of positioning and posture of the left and right legs and feet. The data - angle, pressure exerted, etc., measured using the platform (1') and the camera (2) are then transmitted to a diagnostic processor (3') which in turn sends the diagnosis to a second processor (4'), which is a therapeutic processor. The values calculated by the latter (4') will then be transmitted to the tilting platform (5') for the rehabilitation of the patient (11').

The measured data are transmitted by the "instruments" - platforms 1 and 1' and camera 2, preferably as represented in the diagram - via a bluetooth wireless system; in other words, the data are transmitted to the diagnostic devices not by wires but by radio waves or equivalent systems (infrared etc.) This system improves the precision of the measurement of the parameters for the patient and also makes it possible to utilize the platform at 360° and not, as traditionally up to now, at 180°.

By combining a baropodmetric-stabilometric platform for diagnostics and the further implementation of the values provided by the cameras (2), positioned at right-angles to each other as shown in figure 1, it is possible to determine with the maximum precision the patient's initial postural parameters and to give a precise therapeutic indication, as shown in figure 1. Thus, thanks to this new combination of measurement systems, i.e. the combined use of the baropodometric-stabilometric platform and photographic data for diagnosis and the proprioceptive platform for therapy, more precise data can be obtained as regards both the evaluation of the pathology to be treated, i.e. the real origin of the pathology (which may be dental, ocular, traumatic etc.) as well as in the application of the therapy. In addition to the benefits outlined above, there is the added and significant advantage that traditional systems cannot provide: it will be possible to verify, at successive examinations, the improvements in the patient (11 and 11') by overlaying the photograph taken prior to the commencement of the treatment over the photograph taken after a cycle of treatments or even a single treatment. Comparison of the photographs will make it possible to ascertain the progressive improvement brought about by rehabilitation. The photographic system of measurement, it bears repeating, also makes it possible to obtain data concerning the patient's alignment, angles, vertical and horizontal lines and all other parameters that are necessary for correct alignment of the patient, initially for diagnosis and therapy and subsequently during treatment until the correct posture is achieved and thus the patient is correctly aligned. In addition, the system makes it possible to evaluate the importance and priorities for the treatments to be applied for the various pathologies (i.e. a dental pathology might take priority over an optical pathology).

The importance of this system, therefore, is again underlined; different instruments, which are generally used separately, are used in conjunction in a single situation; in addition, to obtain further improvement, a photographic measurement is added which not only increases the precision of the diagnosis and thus the effectiveness of the treatment, but also to monitor, step by step, the progress achieved and, where necessary, to introduce variations designed to further improve the effectiveness of the treatment.

It should be noted that the specific schematic drawings to which reference is made may be subject to variations though without altering the structural and functional principles described above; for example, personal computers with the appropriate diagnostic and therapeutic software may be used instead of the diagnostic and therapeutic processors 3, 4, 3' and 4' and would perform the same functions; similarly, therapeutic processors 4 and 4' may comprise a single unit or instrument, appropriately calibrated, adjusted or programmed, for diagnostic and therapeutic processing, and are also envisaged.

It is important to clarify that proprioceptive therapeutic platform 5 is the same as proprioceptive therapeutic platform 5', and that patient 11 is the same as patient 11"; in addition, steps 11" of patient 11' are the same as those of patient 11.

Also important is the fact that this system may be used not only for traditional therapeutic functions focusing on physiotherapy and rehabilitation consequent to a trauma etc., but may also be used with success for the treatment and/or prevention of major pathologies - dental, oculistic/optometric, sports (training) - and equally in the field of aesthetic treatments.

It is easily understood that these and other variants may be introduced without altering the principle on which the system is based and claimed herein, and thus without compromising the protection of this industrial patent.

## Claims

1. Improved system for diagnostic determination and consequent treatment of various pathologies by combining postural pression and photographic relevation relevation means **characterized by** the fact that it comprises a "baropodometric-stabilometric" platform (1) comprising the union of two platforms, i.e. a baropodometric platform and a stabilometric platform, as well as photographic cameras (2) positioned at right-angles, for frontal and lateral images of the patient (11), for static measurement of the data relating to the pathology of the patient (11), also **characterized by** the fact that the data measured by the "baropodometric-stabilometric" platform (1) and the photographic cameras (2) are then processed by a diagnostic processor (3) and then transmitted to the therapeutic elaborator (4), which then transmits the therapy to a "proprioceptive platform" (5) for the therapy.

2. System, as described in claim 1, **characterized by** the fact that, in addition to static measurements, by using platform 1' and camera 2, it is possible to make a dynamic measurement of the steps (11") taken by the patient (11), these data being processed by a diagnostic processor (3') and then transmitted to a therapeutic processor (4') and then to a "proprioceptive platform" (5) for integration with the therapy defined at a static level.

3. System as described in the preceding claims, **characterized by** the fact that the diagnostic processors (3 and 3') and therapeutic processors (4 and 4') may be separate units or single units, or may consist of a personal computer with the appropriate software.
